(19) Europäisches Patentamt
European Patent Office
Office européen des brevets

(11) EP 0 864 313 A2

(12) **EUROPÄISCHE PATENTANMELDUNG**

(43) Veröffentlichungstag:
**16.09.1998 Patentblatt 1998/38**

(51) Int. Cl.[6]: **A61K 7/00**, A61K 7/42

(21) Anmeldenummer: **98103535.5**

(22) Anmeldetag: **28.02.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **15.03.1997 DE 19710854**

(71) Anmelder:
**Beiersdorf Aktiengesellschaft**
**20245 Hamburg (DE)**

(72) Erfinder:
- **Gers-Barlag, Heinrich, Dr.**
  **25495 Kummerfeld (DE)**
- **Scheel, Oliver, Dr.**
  **22559 Hamburg (DE)**

(54) **Verwendung von Benzophenonen und deren Derivaten gegen die UV-induzierte Zersetzung von Dibenzoylmethan und dessen Derivaten**

(57) Verwendung kosmetisch oder dermatologisch unbedenklicher Substanzen, deren chemische Formel das Strukturmotiv des Benzophenons enthalten, zur Stabilisierung kosmetische oder dermatologisch unbedenklicher Substanzen, deren chemische Formel das Strukturmotiv des Dibenzoylmethans enthalten, gegen die durch UV-Strahlung induzierte Zersetzung.

EP 0 864 313 A2

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Eine bekannte und vorteilhafte Lichtschutzfiltersubstanz ist das 4-(tert.-Butyl)-4'-methoxydibenzoylmethan, welches sich durch die Struktur

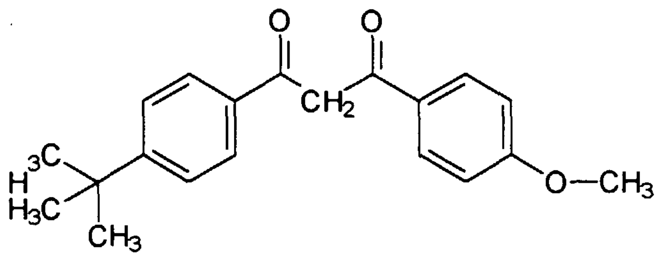

auszeichnet, und von Givaudan unter der Marke Parsol® 1789 verkauft wird

Der Hauptnachteil dieser Substanz ist eine gewisse Instabilität gegenüber UV-Strahlung, so daß es zweckmäßig ist, Zubereitungen mit einem Gehalt an dieser Substanz auch gewisse UV-Stabilisatoren einzuverleiben. Die photochemische Zersetzung von 4-(tert.-Butyl)-4'-methoxydibenzoylmethan - stellvertretend für alle im UV-Bereich absorbierenden Dibenzoylmethanderivate - folgt einer Norrish-Typ-I-Acylspaltung gemäß dem nachfolgenden Reaktionsschema:

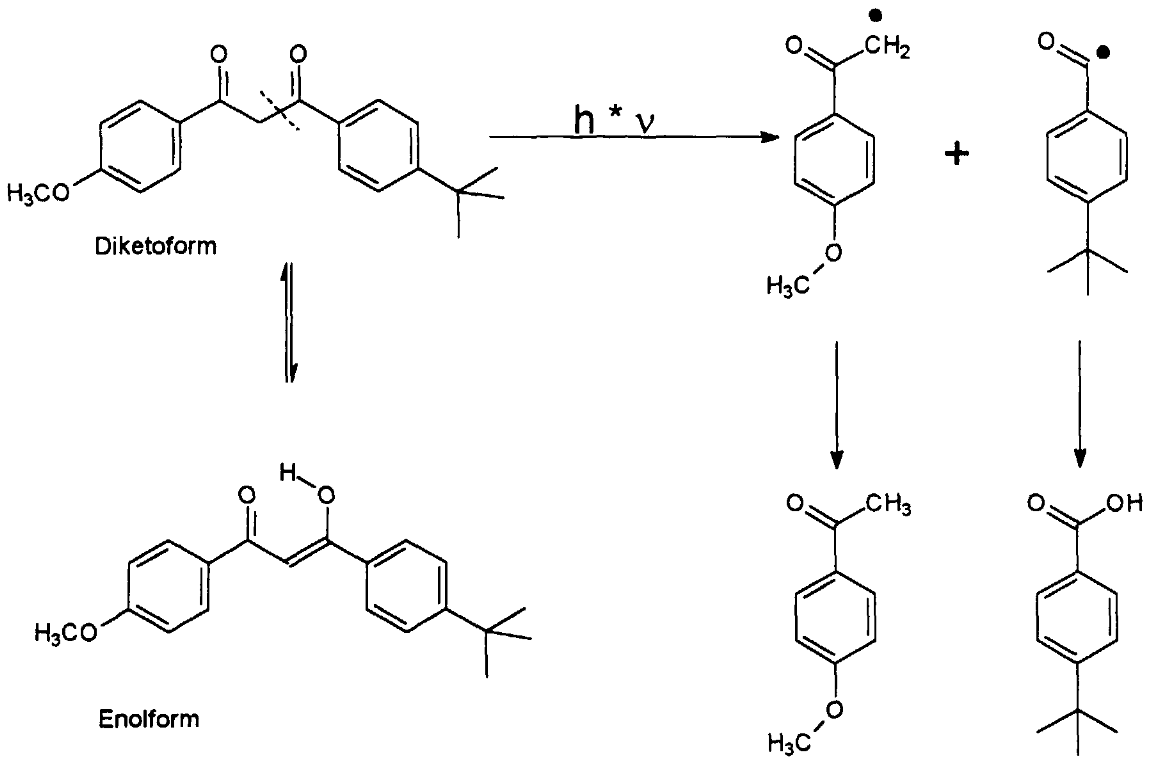


Die Reaktionsprodukte stehen als Lichtschutzfiltersubstanzen nicht mehr zur Verfügung. Es war daher ein dringender Bedarf Wege aufzuweisen, auf welchen der photolytischen Zersetzung von Dibenzoylmethanderivaten wirksam begegnet werden kann.

Beispielsweise beschreibt die deutsche Offenlegungsschrift DE-A-37 41 420 die Kombination dieses Lichtschutzfilters in bestimmtem Mengenverhältnis zu 4-Methylbenzylidencampher, welcher sich durch die Struktur

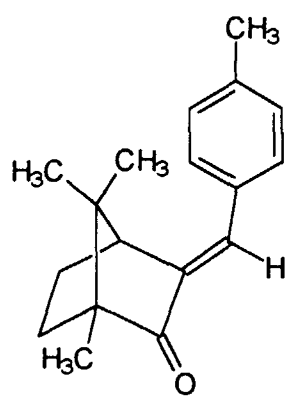

auszeichnet und von Merck unter der Marke Eusolex® 6300 verkauft wird. Die a.a.O. beschriebenen Zubereitungen zeichnen sich aber wiederum durch andere Nachteile aus, hauptsächlich formulierungstechnischer Natur.

Den Nachteilen des Standes der Technik abzuhelfen, war also Aufgabe der vorliegenden Erfindung.

Es war indes überraschend und für den Fachmann nicht vorauszusehen, daß die Verwendung kosmetisch oder dermatologisch unbedenklicher Substanzen, deren chemische Formel das Strukturmotiv des Benzophenons

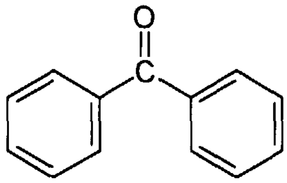

enthalten, zur Stabilisierung kosmetische oder dermatologisch unbedenklicher Substanzen, deren chemische Formel das Strukturmotiv des Dibenzoylmethans

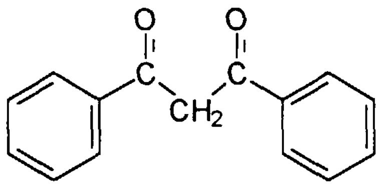

enthalten, gegen die durch UV-Strahlung induzierte Zersetzung, den Nachteilen des Standes der Technik abhelfen.

Liegen Benzophenonderivate in kosmetischen oder dermatologischen Zubereitungen in Kombination mit Dibenzoylmethanderivaten vor, so sind letztere gegen die durch UV-Strahlung induzierte Zersetzung in hervorragender Weise geschützt.

Zwar beschreiben manche Dokumente stabile kosmetische kosmetische Zubereitungen, in denen Dibenzoylmethanderivate vor photochemischer Zersetzung geschützt werden sollen, welche sich durch einen fakultativen Gehalt an Benzophenonderivaten auszeichnen, z.B. WO 94/04131, FR-A-2 687 914, WO 93/02659, DE-A-33 24 735, US-A-5,004,594. Die erfindungsgemäße Lehre wird jedoch von diesen Schritten nicht einmal nahegelegt, denn die Problemlösung geschieht jeweils mit anderen Mitteln.

Vorteilhaft können erfindungsgemäß die Substanzen, die das Strukturmotiv des Benzophenons enthalten (in dieser Schrift auch als „Benzophenone" bezeichnet) gewählt werden aus der Gruppe der folgenden Substanzen

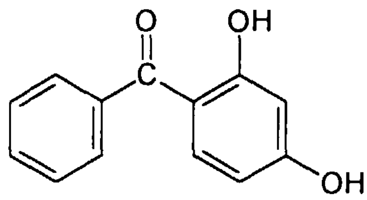

Benzophenon-1
(2,4-Dihydroxybenzophenon)
CAS-Nr. 131-56-6

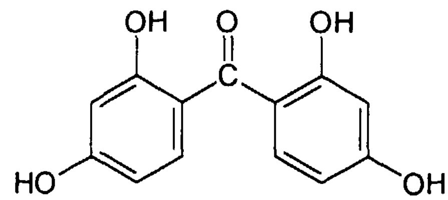

Benzophenon-2
(2,2',4,4'-Tetrahydroxybenzophenon)
CAS-Nr. 131-55-5

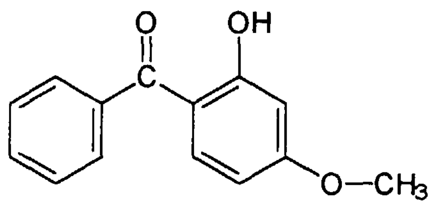

Benzophenon-3
(2-Hydroxy-4-methoxybenzophenon)

CAS-Nr. 131-57-7

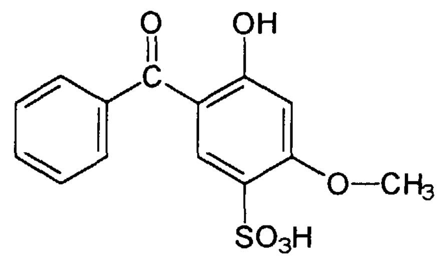

Benzophenon-4
(2-Hydroxy-4-methoxybenzophenon-5-sulfonsäure)
CAS-Nr. 4065-45-6

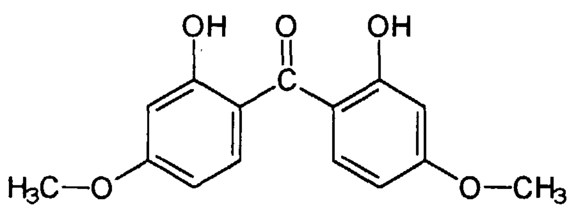

Benzophenon-6
(2,2'-Dihydroxy-4,4'-dimethoxybenzophenon)

CAS-Nr. 131-54-4

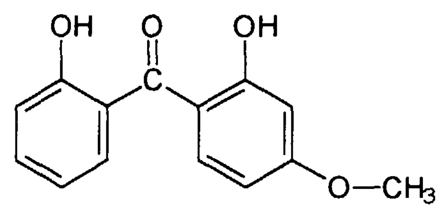

Benzophenon-8
(2,2'-Dihydroxy-4-methoxybenzophenon)
CAS-Nr. 131-53-3

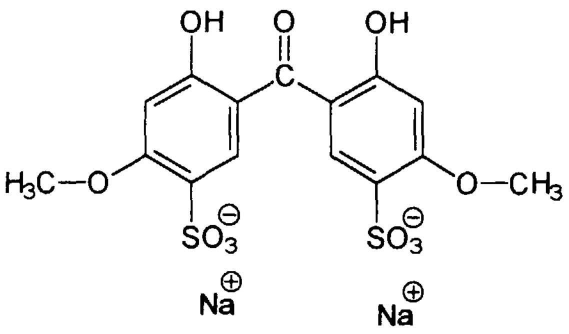

Benzophenon-9

(Dinatrium-2,2'-dihydroxy-4,4'-dimethoxy-5,5'-disulfobenzophenon)

Cas-Nr. 3121-60-6,

ferner Benzophenon-11, CAS-Nr. 1341-54-4.

Bevorzugtes Benzophenon ist Benzophenon-3, welches beispielsweise von Merck unter der Warenbezeichnung Eusolex® 4360 verkauft wird.

Von den Dibenzoylmethanderivaten werden vorteilhaft verwendet:

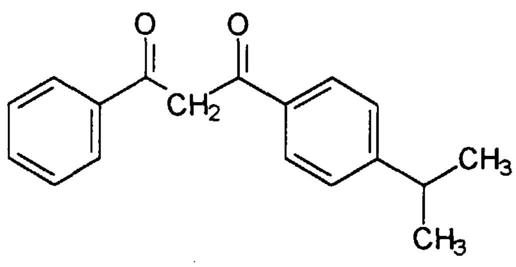

4-Isopropyldibenzoylmethan

CAS-Nr. 63260-25-9

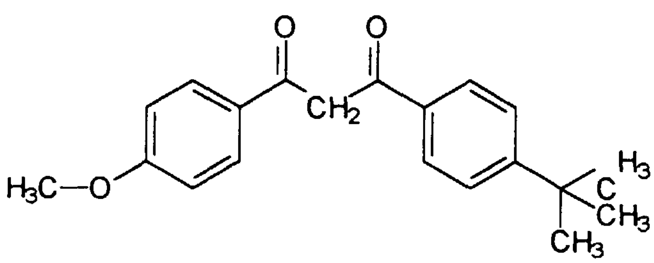

4-(tert.-Butyl)-4'-methoxydibenzoylmethan

CAS-Nr. 70356-09-1

Bei Befolgen der erfindungsgemäßen Lehre sind Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan

gegen die Zersetzung unter UV-Licht wird drastisch erhöht. Ganz besonders erstaunlich war, daß die Erhöhung der Stabilität von Dibenzoylmethanen, insbesondere von 4-(tert.-Butyl)-4'-methoxydibenzoylmethan gleichermaßen erfolgt, wenn diese in polaren aber auch unpolaren Ölkomponenten gelöst vorliegen.

Die Gesamtmenge an Dibenzoylmethanen, insbesondere 4-(tert.-Butyl)-4'-methoxydibenzoylmethan in den fertigen kosmetischen oder dermatologischen Zubereitungen wird vorteilhaft aus dem Bereich von 0,1 - 10,0 Gew.-%, bevorzugt 0,5 - 6,0 Gew.-% gewährt, bezogen auf das Gesamtgewicht der Zubereitungen.

Die Gesamtmenge an Benzophenonen, insbesondere Benzophenon-3, in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Besonders vorteilhaft ist es, Gewichtsverhältnisse von Benzophenonen zu Dibenzoylmethanen wie 8 : 1 bis 1 : 5, bevorzugt wie 4 : 1 bis 1 : 2, besonders bevorzugt wie 3 : 1 bis 1 : 1 zu wählen.

Erfindungsgemäße kosmetische und dermatologische Zubereitungen enthalten außerdem vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Die anorganischen Pigmente liegen erfindungsgemäß in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ \text{(oberfl.)}$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewährt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewährt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewährt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewährt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewährt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten UVB-Filter, die zusätzlich im Sinne der vorliegenden Erfindung eingesetzt werden können, soll selbstverständlich nicht limitierend sein.

Es kann auch von Vorteil sein, UVA-Filter einzusetzen, die bisher üblicherweise in kosmetischen Zubereitungen enthalten sind. Es können die für die UVB-Kombination verwendeten Mengen eingesetzt werden.

Ferner kann gegebenenfalls von Vorteil sein, die erfindungsgemäßen Wirkstoffkombinationen mit weiteren UVA- und/oder UVB-Filtern zu kombinieren, beispielsweise bestimmten Salicylsäurederivaten wie

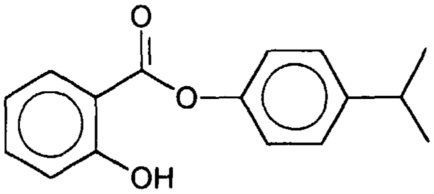

(4-Isopropylbenzylsalicylat),

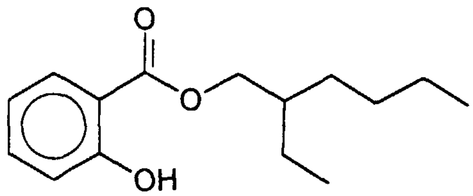

(2-Ethylhexylsalicylat, Octylsalicylat),

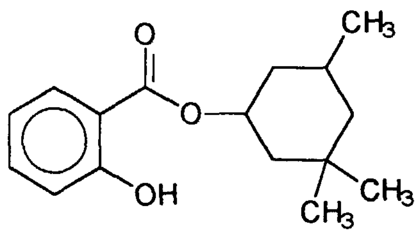

(Homomenthylsalicylat).

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung UVINUL® N 539 erhältlich ist und sich durch folgende Struktur auszeichnet:

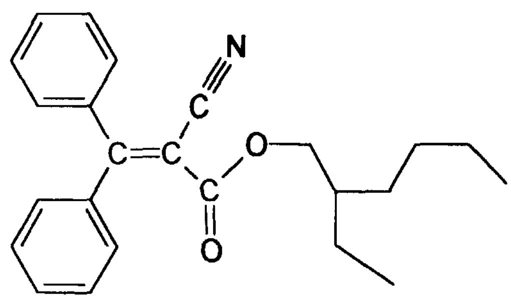

Es hat sich darüberhinaus als vorteilhaft im Sinne der vorliegenden Erfindung herausgestellt, keine zusätzlichen UV-Filtersubstanzen zu verwenden, welche der Gruppe der Zimtsäurederivate angehören. Ferner ist es im Sinne der vorliegenden Erfindung von Vorteil, keine zusätzlichen UV-Filtersubstanzen zu verwenden, welche der Gruppe der der Campherderivate, das sind insbesondere die Derivate des 3-Benzylidencamphers, angehören.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1**<br>O/W-Lotion | |
|---|---|
| | Gew.-% |
| Glycerylstearat | 3,50 |
| Stearinsäure | 1,80 |
| Glycerin | 3,00 |
| Cetylstearylalkohol | 0,50 |
| Natriumhydroxid (45 %ig) | 0,20 |
| Octyldodecanol | 7,00 |
| Dicaprylether | 8,00 |
| 2,4,6-Trianilino-(p-carbo-2'-ethyl-1'-hexyloxy)-1,3,5-triazin | 3,00 |
| Benzophenon 3 | 3,00 |
| 4-(tert.-Butyl)-4'-methoxydibenzoyl-methan | 2,00 |
| Heptansäure-2.2-dimethyl-1.3-propandioldiester | 6,00 |
| Carbomer | 0,20 |
| Konservierungsmittel | q.s. |
| Parfum | q.s. |
| Wasser, demin. | ad 100,00 |

| **Beispiel 2**<br>Hydrodispersionsgel | |
|---|---|
| | Gew.-% |
| Pemulen TR-1 | 0,50 |
| Ethanol | 3,50 |
| Glycerin | 3,00 |
| Dimethicon | 1,50 |
| Natriumhydroxid (45 %ig) | 0,55 |
| Octyldodecanol | 0,50 |
| Capric/Caprylic Triglyceride | 5,00 |
| Heptansäure-2.2-dimethyl-1.3-propandiolester | 5,00 |
| Octocrylen | 10,00 |
| Benzophenon 3 | 5,00 |
| 4-(tert.-Butyl)-4'-methoxydibenzoyl-methan | 4,00 |
| Carbomer | 0,20 |
| Konservierungsmittel | q.s. |
| Parfum | q.s. |

(fortgesetzt)

| **Beispiel 2** Hydrodispersionsgel | |
|---|---|
| | Gew.-% |
| Wasser, demin. | ad 100,00 |

| **Beispiel 3** O/W-Creme | |
|---|---|
| | Gew.-% |
| Glycerylstearat SE | 3,50 |
| Stearinsäure | 3,50 |
| Butylenglykol | 5,00 |
| Cetylstearylalkohol | 3,00 |
| Natriumhydroxid (45 %ig) | 0,35 |
| $C_{12}$-$C_{15}$ Alkylbenzoate | 10,00 |
| 2,4,6-Trianilin-(p-carbo-2'-ethyl-1'-hexyloxy)-1,3,5-triazin | 4,00 |
| Benzophenon 3 | 5,00 |
| 4-(tert.-Butyl)-4'-methoxydibenzoyl-methan | 2,00 |
| $TiO_2$ | 3,00 |
| Octyldodecanol | 6,00 |
| Carbomer | 0,20 |
| Konservierungsmittel | q.s. |
| Parfum | q.s. |
| Wasser, demin. | ad 100,00 |

| **Beispiel 4** W/O-Lotion | |
|---|---|
| | Gew.-% |
| Polyglyceryl-2-Polyhydroxystearat | 3,50 |
| Polyglyceryl-3-Diisostearat | 3,50 |
| Butylenglykol | 5,00 |
| Ceresin | 3,00 |
| Natriumhydroxid (45 %ig) | 0,35 |
| $C_{12}$-$C_{15}$ Alkylbenzoate | 10,00 |
| Octocrylen | 10,00 |

(fortgesetzt)

| **Beispiel 4** W/O-Lotion | |
|---|---|
| | Gew.-% |
| Benzophenon 3 | 2,00 |
| 4-(tert.-Butyl)-4'-methoxydibenzoyl-methan | 1,00 |
| Eusolex 232 | 2,00 |
| NaOH | q.s. |
| Miglyol 812 | 6,00 |
| Vaseline | 2,00 |
| Konservierungsmittel | q.s. |
| Parfum | q.s. |
| Wasser, demin. | ad 100,00 |

| **Beispiel 5** O/W-Lotion | |
|---|---|
| | Gew.-% |
| Glycerylstearat | 3,50 |
| Stearinsäure | 1,80 |
| Glycerin | 3,00 |
| Cetylstearylalkohol | 0,50 |
| Natriumhydroxid (45 %ig) | 0,20 |
| Octyldodecanol | 7,00 |
| Dicaprylylether | 8,00 |
| 2,4,6-Trianilino-(p-carbo-2'-ethyl-1'-hexyloxy)-1,3,5-triazin | 3,00 |
| $TiO_2$ (hydrophob) | 2,00 |
| 4-(tert.-Butyl)-4'-methoxydibenzoyl-methan | 2,00 |
| Benzophenon 3 | 4,00 |
| Tricaprylin | 6,00 |
| Carbomer | 0,20 |
| Konservierungsmittel | q.s. |
| Parfum | q.s. |
| Wasser, demin. | ad 100,00 |

| **Beispiel 6**<br>Hydrodispersionsgel | |
|---|---|
| | Gew.-% |
| Pemulen TR-1 | 0,50 |
| Ethanol | 3,50 |
| Glycerin | 3,00 |
| Dimethicon | 1,50 |
| Natriumhydroxid (45 %ig) | 0,55 |
| Octyldodecanol | 0,50 |
| Capric/Caprylic Triglyceride | 5,00 |
| Arlasolv DM | 5,00 |
| 2,4,6-Trianilino-(p-carbo-2'-ethyl-1'-hexyloxy)-1,3,5-triazin | 5,00 |
| Benzophenon 3 | 3,00 |
| $TiO_2$ (hydrophob) | 2,00 |
| 4-(tert.-Butyl)-4'-methoxydibenzoyl-methan | 2,00 |
| Carbomer | 0,20 |
| Konservierungsmittel | q.s. |
| Parfum | q.s. |
| Wasser, demin. | ad 100,00 |

| **Beispiel 7**<br>O/W-Creme | |
|---|---|
| | Gew.-% |
| Glycerylstearat SE | 3,50 |
| Stearinsäure | 3,50 |
| Butylenglykol | 5,00 |
| Cetylstearylalkohol | 3,00 |
| Natriumhydroxid (45 %ig) | 0,35 |
| $C_{12}$-$C_{15}$ Alkylbenzoate | 10,00 |
| 2,4,6-Trianilino-(p-carbo-2'-ethyl-1'-hexyloxy)-1,3,5-triazin | 4,00 |
| $TiO_2$ (hydrophob) | 2,00 |
| 4-(tert.-Butyl)-4'-methoxydibenzoyl-methan | 2,00 |
| Cosmacolen | 6,00 |
| Benzophenon 3 | 4,00 |
| Carbomer | 0,20 |

(fortgesetzt)

| **Beispiel 7** O/W-Creme | |
|---|---|
| | Gew.-% |
| Konservierungsmittel | q.s. |
| Parfum | q.s. |
| Wasser, demin. | ad 100,00 |

| **Beispiel 8** W/O-Lotion | |
|---|---|
| | Gew.-% |
| Polyglyceryl-2-Polyhydroxystearat | 3,50 |
| Polyglyceryl-3-Diisostearat | 3,50 |
| Butylenglykol | 5,00 |
| Ceresin | 3,00 |
| Natriumhydroxid (45 %ig) | 0,35 |
| $C_{12}$-$C_{15}$ Alkylbenzoate | 10,00 |
| 2,4,6-Trianilino-(p-carbo-2'-ethyl-1'-hexyloxy)-1,3,5-triazin | 4,00 |
| $TiO_2$(hydrophob) | 2,00 |
| 4-(tert.-Butyl)-4'-methoxydibenzoyl-methan | 2,00 |
| Cosmacolen | 6,00 |
| Benzophenon 3 | 5,00 |
| Vaseline | 2,00 |
| Konservierungsmittel | q.s. |
| Parfum | q.s. |
| Wasser, demin. | ad 100,00 |

## Patentansprüche

1. Verwendung kosmetisch oder dermatologisch unbedenklicher Substanzen, deren chemische Formel das Strukturmotiv des Benzophenons

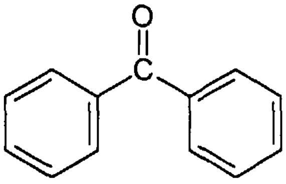

enthalten, zur Stabilisierung kosmetische oder dermatologisch unbedenklicher Substanzen, deren chemische Formel das Strukturmotiv des Dibenzoylmethans

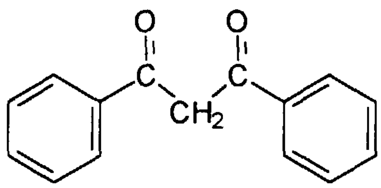

enthalten, gegen die durch UV-Strahlung induzierte Zersetzung.

2. Verwendung nach Anspruch 1, dadurch gekennzeichnet, daß die Substanzen, die das Strukturmotiv des Benzophenons aufweisen, gewährt werden aus der Gruppe Benzophenon-1, Benzophenon-2, Benzophenon-3, Benzophenon-4,Benzophenon-6, Benzophenon-8, Benzophenon-9, Benzophenon-11.

3. Verwendung, nach Anspruch 1, dadurch gekennzeichnet, daß die Substanzen, die das Strukturmotiv des Dibenzoylmethans aufweisen, gewählt werden aus der Gruppe 5-Isopropyldibenzoylmethan, 4-(tert.-Butyl)-4'-methoxydibenzoylmethan.

4. Verwendung nach Anspruch 1, dadurch gekennzeichnet, daß die Gesamtmenge an Substanzen, die das Strukturmotiv des Dibenzoylmethans aufweisen, insbesondere 4-(tert.-Butyl)-4'-methoxydibenzoylmethan, in den fertigen kosmetischen oder dermatologischen Zubereitungen aus dem Bereich von 0,1 - 10,0 Gew.-%, bevorzugt 0,5 - 6,0 Gew.-%, bezogen auf das Gesamtgewicht der Zubereitungen gewählt wird.

5. Verwendung nach Anspruch 1, dadurch gekennzeichnet, daß die Gesamtmenge an Substanzen, die das Strukturmotiv des Benzophenons aufweisen, insbesondere Benzophenon-3, in den fertigen kosmetischen oder dermatologischen Zubereitungen aus dem Bereich von 0,1 - 10,0 Gew.-%, bevorzugt 0,5 - 6,0 Gew.-%, bezogen auf das Gesamtgewicht der Zubereitungen gewählt wird.

6. Verwendung nach Anspruch 1, dadurch gekennzeichnet, daß die Gewichtsverhältnisse der Substanzen, die das Strukturmotiv des Benzophenons aufweisen, zu den Substanzen, die das Strukturmotiv des Dibenzoylmethans aufweisen, wie 8 : 1 bis 1 : 5, bevorzugt wie 4 : 1 bis 1 : 2, besonders bevorzugt wie 3 : 1 bis 1 : 1 gewählt werden.

7. Verwendung nach Anspruch 1 in kosmetischen Lichtschutzzubereitungen, welche keine UV-Filtersubstanzen enthalten, die der Gruppe der Zimtsäurederivate angehören.